# FASCICULE DE BREVET EUROPEEN

(11) **EP 1 281 760 B1**
(45) Date de publication et mention de la délivrance du brevet: **13.07.2005**
(21) Numéro de dépôt: 02017134.4
(22) Date de dépôt: 01.10.1998
(51) Int. Cl.: C12N 15/34, C07K 14/01, A61K 39/12, A61K 48/00, C12Q 1/68, C07K 16/08, G01N 33/53

(54) **Circovirus porcins**
Schweinecircoviren
Porcine circoviruses

(30) Priorité: 03.10.1997 FR 9712382; 22.01.1998 FR 9800873; 20.03.1998 FR 9803707
(43) Date de publication de la demande: 05.02.2003
(62) Demande divisionnaire de: 98946547.1
(73) Titulaire: MERIAL, 69002 Lyon (FR); The Queen's University of Belfast, Belfast BT4 3SD (GB); The University of Saskatchewan, Saskatoon, Saskatchewan S7W 5B4 (CA)
(72) Inventeur: Allan, Gordon, Belfast BT5 7AQ (GB); Meehan, Brian, Belfast BT1 3LX (GB); Clark, Edward, Saskatoon, Saskatchewan S7 J214 (CA); Ellis, John, Saskatoon, Saskatchewan S7NOM3 (CA); Haines, Deborah, Saskatoon, Saskatchewan S7NOM3 (CA); Hassard, Lori, Saskatoon, Saskatchewan S7K2A0 (CA); Harding, John, Saskatchewan 2 SOK 2AO (CA); Charreyre, Catherine Elisabeth, 69720 Saint-Laurent de Mure (FR); Chappuis, Gilles Emile, 69006 Lyon (FR); Mc Neilly, Francis, Newtownards BT3 4NH (GB)
(74) Mandataire: Nargolwalla, Cyra

(56) Documents cités:
- WO-A-96/06619
- WO-A-99/29717
- NAYAR G.P.S. ET AL.: "Detection and characterization of porcine circovirus associated with postweaning multisystemic wasting syndrome in pigs" CANADIAN VETERINARY JOURNAL - REVUE VETERINAIRE CANADIENNE, vol. 38, juin 1997 (1997-06), pages 385-386, XP002068396
- CLARK E. G.: "Post-weaning multisystemic wasting syndrome" PROCEEDINGS OF THE AMERICAN ASSOCIATION OF SWINE PRACTITIONERS. ANNUAL MEETING, 1 mars 1997 (1997-03-01), pages 499-501, XP002068397
- MEEHAN B.M. ET AL.: "Characterization of novel circovirus DNAs associated with wasting syndromes in pigs" JOURNAL OF GENERAL VIROLOGY, vol. 79, no. 9, septembre 1998 (1998-09), pages 2171-2179, XP002090386
- HAMEL A.L. ET AL.: "Nucleotide sequence of Porcine Circovirus associated with postweaning multisystemic wasting syndrome in pigs" JOURNAL OF VIROLOGY, vol. 72, no. 6, juin 1998 (1998-06), pages 5262-5267, XP002078783
- MOROZOV I. ET AL.: "Detection of a novel strain of porcine circovirus in pigs with postweaning multisystemic wasting syndrome" JOURNAL OF CLINICAL MICROBIOLOGY, vol. 36, no. 9, septembre 1998 (1998-09), pages 2535-2541, XP002090921
- ELLIS J ET AL: "ISOLATION OF CIRCOVIRUS FROM LESIONS OF PIGS WITH POSTWEANING MULTISYSTEMIC WASTING SYNDROME" CANADIAN VETERINARY JOURNAL - REVUE VETERINAIRE CANADIENNE, OTTAWA, CA, vol. 39, 1998, pages 44-51, XP002068502 ISSN: 0008-5286
- ALLAN G M ET AL: "ISOLATION OF PORCINE CIRCOVIRUS-LIKE VIRUSES FROM PIGS WITH A WASTING DISEASE IN THE USA AND EUROPE" JOURNAL OF VETERINARY DIAGNOSTIC INVESTIGATION, AAVLD, COLUMBIA, MO, US, vol. 10, 1998, pages 3-10, XP002068503 ISSN: 1040-6387
- SEGALES J ET AL: "FIRST REPORT OF POST-WEANING MULTISYSTEMIC WASTING SYNDROME IN PIGS IN SPAIN" VETERINARY RECORD, LONDON, GB, vol. 141, no. 23, 6 décembre 1997 (1997-12-06), pages 600-601, XP002068504 ISSN: 0042-4900
- MEEHAN B.M. ET AL.: "Sequence of porcine circovirus DNA: affinities with plant circoviruses" JOURNAL OF GENERAL VIROLOGY, vol. 78, no. 1, janvier 1997 (1997-01), pages 221-227, XP002068398
- MANKERTZ A. ET AL.: "Mapping and characterization of the origin of DNA replication of porcine circovirus" JOURNAL OF VIROLOGY, vol. 71, no. 3, mars 1997 (1997-03), pages 2562-2566, XP002078782
- TODD D ET AL: "COMPARISON OF THREE ANIMAL VIRUSES WITH CIRCULAR SINGLE-STRANDED DNA GENOMES" ARCHIVES OF VIROLOGY, NEW YORK, NY, US, vol. 117, 1991, pages 129-135, XP002068399 ISSN: 0304-8608

## Description

La présente invention est relative à de nouvelles souches et préparations de circovirus porcin (PCV pour *Porcine Circo Virus*) responsables du syndrome PMWS (*Porcine Multisystemic Wasting Syndrome* ou *Post-Weaning Multisystemic Wasting Syndrome* encore appelé syndrome de dépérissement généralisé de post-sevrage), ainsi qu'à des méthodes de production de ces circovirus.

Le PCV a été à l'origine détecté comme contaminant non cytopathogène dans des lignées cellulaires de reins de porcs PK/16. Ce virus a été classé parmi les Circoviridae avec le virus de l'anémie du poulet (CAV pour *Chicken Anemia Virus*) et le virus PBFDV (*Pscittacine Beak and Feather Disease Virus*). Il s'agit de petits virus (de 16 à 24 nm) non enveloppés dont la caractéristique commune est de contenir un génome sous forme d'un ADN simple brin circulaire de 1,76 à 2,31 kb. On a d'abord pensé que ce génome codait pour un polypeptide d'environ 30 kDa (Todd et al., Arch Virol 1991, 117: 129-135). Des travaux récents ont toutefois montré une transcription plus complexe (Meehan B. M. et al., 1997, 78: 221-227). Par ailleurs, on ne connaît pas d'homologies significatives de séquence nucléotidique ni de déterminants antigéniques communs entre les trois types de circovirus connus.

Le PCV issu des cellules PK/16 est considéré comme n'étant pas pathogène. On en connaît la séquence d'après B. M. Meehan et al., J Gen Virol 1997 (78) 221-227. Ce n'est que très récemment que des auteurs ont pensé que des souches de PCV pourraient être pathogènes et associées au syndrome PMWS (Gupi P. S. Nayar et al., Can Vet J , vol. 38, 1997: 385-387 et Clark E. G., Proc Am Assoc Swine Prac 1997: 499-501). Nayar et al. ont détecté de l'ADN de PCV chez des porcs présentant le syndrome PMWS par des techniques de PCR. Aucune souche sauvage de PCV n'a toutefois été isolée et purifiée à ce jour.

Le syndrome PMWS détecté au Canada, aux Etats-Unis et en France se caractérise au plan clinique par une perte progressive de poids et par des manifestations telles que tachypnée, dyspnée et jaunisse. Au plan pathologique, il se traduit par des infiltrations lymphocytaires ou granulomateuses, des tymphadénopathies et, plus rarement, par des hépatites et néphrites lymphocytaires ou granulomateuses (Clark E. G., Proc. Am. Assoc. Swine Prac. 1997: 499-501 ; La Semaine Vétérinaire n° 26, supplément à La Semaine Vétérinaire 1996 (834) ; La Semaine Vétérinaire 1997 (857): 54 ; Gupi P. S. Nayar et al., Can Vet J , vol. 38, 1997: 386-387).

La déposante a réussi à isoler cinq souches nouvelles de PCV à partir de prélèvements pulmonaires ou ganglionnaires provenant d'élevages situés au Canada, aux Etats-Unis (Californie) et en France (Bretagne), ci-après dénommés circovirus selon l'invention. Ces virus ont été mis en évidence dans des lésions de porcs atteints du syndrome PMWS, mais pas chez des porcs sains.

La déposante a en outre séquencé le génome de quatre de ces souches, à savoir les souches provenant du Canada et des Etats-Unis ainsi que deux souches française. Les souches présentent entre elles une très forte homologie au niveau nucléotidique dépassant 96 % et beaucoup plus faible avec la souche PK/16, environ 76 %. Les nouvelles souches peuvent donc être considérées comme représentatives d'un nouveau type de circovirus porcin, dénommé ici type II, le type I étant représenté par la PK/15.

La présente invention a donc pour objet le circovirus porcin de groupe II, tel que défini ci-dessus, isolé ou sous forme de préparation purifiée.

L'invention concerne tout circovirus porcin susceptible d'être isolé d'un échantillon physiologique ou d'un prélèvement tissulaire, notamment de lésions, d'un porc malade présentant le syndrôme PMWS, notamment en suivant la méthode décrite dans les exemples, en particulier circovirus du type II.

La présente invention a plus particulièrement pour objet des préparations purifiées de cinq souches, qui ont été déposées auprès de l'ECACC (European Collection of Cell Cultures, Centre for Applied Microbiology & Research, Porton Down, Salisbury, Wiltshire SP4 OJG, Royaume-Uni) le jeudi 2 octobre 1997:
- n° d'accès V97100219 (appelé ici Imp.1008PCV)
- n° d'accès V97100218 (appelé ici lmp.1010PCV)
- n° d'accès V97100217 (appelé ici Imp.999PCV).
   et, le vendredis16 janvier1998 :
- n° d'accès V98 011608 (appelé ici Imp 1011-48285)
- n° d'accès V98 011609 (appelé ici lmp 1011-48121)

L'invention entend considérer les circovirus porcins isolés d'un porc malade et/ou les circovirus ayant une parenté sérologique significative avec les souches de l'invention et/ou les circovirus ayant une hybridation croisée avec les souches de l'invention dans des conditions de stringence telles qu'il n'y a pas d'hybridation avec la souche PCV PK/15.

Les souches virales isolées d'un échantillon physiologique ou d'un prélèvement tissulaire, notamment d'une lésion, d'un porc présentant le syndrome PMWS peuvent être avantageusement propagées sur des lignées cellulaires telles que notamment des lignées cellulaires de rein de porc, en particulier cellules PK/15 indemnes de contamination (en particulier pour PCV, ainsi que pour pestivirus, adénovirus porcin et parvovirus porcin) en vue de leur multiplication ou spécifiquement pour la production d'antigène, entier (e.g. virus) et/ou sous-unités (e.g. polypeptides).

De manière très remarquable et inattendue, ces isolats se sont révélés très productifs en culture sur cellules PK/15, ce qui présente des avantages indéniables pour la production de virus ou d'antigène, en particulier pour le production de vaccin inactivé.

La présente invention a aussi pour objet les préparations de circovirus isolés après passages sur cellules, notamment lignées cellulaires, e.g. cellules PK/16, cultivées in vitro en étant infectées par l'un au moins des circovirus selon l'invention ou de tout circovirus porcin susceptible d'être isolé d'un échantillon physiologique ou d'un prélèvement tissulaire, notamment de lésions, d'un porc présentant le syndrôme PMWS. Elle a aussi pour objet les surnageants ou extraits de culture, éventuellement purifiés par des techniques standards, et de manière générale toute préparation antigénique obtenue à partir des cultures in vitro.

Les circovinn selon l'invention, avec les fractions qui peuvent être présentes, sont inactivés selon les techniques connues de l'homme du métier. L'inactivation sera effectuée de préférence par voie chimique, e.g. par exposition de l'antigène à un agent chimique tel que formaldéhyde (formol), paraformaldéhyde, β-propiolactone ou éthylène imine ou ses dérivés. La méthode d'inactivation préférée sera ici l'exposition à un agent chimique et en particulier à l'éthylène imine ou à la β-propiolactone.

La déposante a en outre obtenu le génome de quatre des isolats, identifiés SEQ ID NO: 1 à 4 et éventuellement 6.

La présente invention fournit donc un fragment d'ADN contenant tout ou partie de l'une de ces séquences. Il va de soi que l'invention recouvre automatiquement les séquences équivalentes, c'est-à-dire les séquences ne changeant pas la fonctionnalité ni la spécificité de souche de la séquence décrite ni des polypeptides codés par cette séquence. Seront bien entendu incluses les séquences différant par dégénérescence du code.

L'invention recouvre également les séquences équivalentes en ce sens qu'elles sont capables de s'hybrider à la séquence supra dans des conditions de stringence élevées et/ou ont une forte homologie avec les souches de l'invention et appartiennent au groupe II défini plus haut.

L'invention va être maintenant décrite plus en détail à l'aide d'exemples de réalisation non limitatifs, pris en référence au dessin, dans lequel :
**Figure 1** : séquence d'ADN du génome de la souche lmp 1011-48121
**Figure 2** : séquence d'ADN du génome de la souche Imp 1011-48285
**Figure 3** : séquence d'ADN du génome de la souche lmp 999
**Figure 4** : séquence d'ADN du génome de la souche lmp 1010
**Figure 5** : alignement des 4 séquences selon les figures 1 à 4 avec la séquence de la souche PCV PK/15
**Figure 6** : séquence d'ADN du génome de la souche lmp 999 telle que définie dans le premier dépôt en France du 3 octobre 1997
**Figure 7** : Alignements de la séquence de la figure 6 avec la séquence de la souche PK/15

### Liste des séquences SEQ ID

- **SEQ ID NO: 1**: séquence d'ADN du génome de la souche lmp 1011-48121
- **SEQ ID NO: 2**: séquence d'ADN du génome de la souche lmp 1011-48285
- **SEQ ID NO: 3**: séquence d'ADN du génome de la souche lmp 999
- **SEQ ID NO: 4**: séquence d'ADN du génome de la souche lmp 1010
- **SEQ ID NO: 5**: séquence d'ADN du génome de la souche PK/16
- **SEQ ID NO : 6**: séquence d'ADN du génome de la souche lmp 999 telle que définie dans le premier dépôt en France du 3 octobre 1997.

### EXEMPLES

### Exemple 1 : Culture et isolement des souches de circovirus porcins:

Des échantillons de tissus ont été récoltés en France, au Canada et aux USA à partir de poumons et de ganglions lymphatiques de porcelets. Ces porcelets présentaient des signes cliniques typiques du syndrome de dépérissement généralisé de post-sevrage. Pour faciliter l'isolement des virus, les échantillons de tissus ont été congelés à -70°C immédiatement après autopsie.

Pour l'isolement viral, des suspensions contenant environ 16% d'échantillon de tissu ont été préparées dans un milieu minimum contenant des sels d'Earl (EMEM, BioWhittaker UK Ltd., Wokingham, UK), de la pénicilline (100 Ul/ml) et de la streptomycine (100 µg/ml)(milieu MEM-SA), par broyage des tissus avec du sable stérile au moyen d'un mortier et d'un pilon stériles. Cette préparation broyée a été alors reprise dans du MEM-SA, puis centrifugée à 3000 g pendant 30 minutes à + 4°C pour récolter le surnageant.

Préalablement à l'ensemencement des cultures de cellules, un volume de 100 µl de chloroforme a été ajouté à 2 ml de chaque surnageant et mélangé en continu pendant 10 minutes à température ambiante. Ce mélange a alors été transféré dans un tube de microcentrifugeuse, centrifugé à 3000 g pendant 10 minutes, puis le surnageant a été récolté. Ce surnageant a été ensuite utilisé comme inoculum pour les expériences d'isolement viral.

Toutes les études d'isolement viral ont été réalisées sur des cultures de cellules PK/15, connues pour être non contaminées par le circovirus porcin (PCV), les pestivirus, les adénovirus porcins et le parvovirus porcin (Allan G. *et al*. Pathogenesis of porcine circovirus experimental infections of colostrum-deprived piglets and examination of pig foetal material. Vet. Microbiol. 1995. 44. 49-64).

L'isolement des circovirus porcins a été réalisé selon la technique suivante:

Des monocouches de cellules PK/15 ont été dissociées par trypsination (avec un mélange trypsine-versène) à partir de cultures confluentes, et reprises en milieu MEM-SA contenant 15% de sérum foetal de veau non contaminé par du pestivirus (= milieu MEM-G) sous une concentration finale d'environ 400,000 cellules par ml. Des fractions aliquotes de 10 ml de cette suspension cellulaire ont alors été mélangées avec des fractions aliquotes de 2 ml des inoculums décrits ci-dessus, et les mélanges finaux ont été aliquotés en volumes de 6 ml dans deux flacons Falcon de 26 cm². Ces cultures ont alors été incubées à +37°C pendant 18 heures en atmosphère contenant 10% de CO₂.

Après Incubation, le milieu de culture des monocouches semi-confluentes a été traité avec 300 mM de D-glucosamine (Cat # G48176, Sigma-Aldrich Company Limited, Poole, UK) (Tischr I. et al., Arch. Virol. 1987 96 39-57), puis l'incubation a été poursuivie pendant une période supplémentaire de 48-72 heures à +37°C. A la suite de cette dernière incubation, l'un des deux Falcons de chaque inoculum a subi 3 cycles successifs de congélation/décongélation. Les cellules PK/15 du Falcon restant ont été traitées avec une solution de trypsine-versène, resuspendues dans 20 ml de milieu MEM-G, puis ensemencées dans des Falcons de 75 cm² à une concentration de 400,000 cellules/ml. Les flacons fraîchement ensemencés ont alors été "surinfectés" par addition de 5 ml du lysat correspondant obtenu après les cycles de congélation/décongélation.

### Exemple 2 : Préparation des échantillons de culture cellulaire pour détection des circovirus porcins par immunofluorescence ou par hybridation in situ.

Un volume de 5 ml de la suspension "surinfectée" a été prélevé et ensemencé dans une boîte de Pétri de 55 mm de diamètre contenant une lamelle de verre stérile et dégraissée. Les cultures en flacons et sur lamelles de verre ont été incubées à + 37°C et traitées à la glucosamine comme décrit dans l'exemple 1. Les cultures sur lamelles de verre ont été récoltées de 24 à 48 heures après le traitement à la glucosamine et fixées, soit avec de l'acétone pendant 10 minutes à température ambiante, soit avec 10% de formaldéhyde tamponné pendant 4 heures. Suite à cette fixation, toutes les lamelles de verre ont été stockées à -70°C, sur gel de silice, avant leur utilisation pour les études d'hybridation *in situ* et les études de marquage immunocytochimique.

### Exemple 3 : Techniques de détection de séquences PCV par hybridation in situ

L'hybridation *in situ* a été réalisée sur les tissus prélevés sur les porcs malades et fixés au formaldéhyde et également sur les préparations de cultures de cellules inoculées pour l'isolement viral (voir exemple 2) et fixées sur lamelles de verre.

Des sondes génomiques complètes correspondant aux circovirus porcin PK/15 (PCV) et au virus de l'anémie infectieuse du poulet (chicken anemia virus = CAV) ont été utilisées. Le plasmide pPCV1, contenant la forme réplicative du génome PCV clonée sous la forme d'un insert unique de 1,7 kilopaires de bases (kpb) (Meehan B. *et al*. Sequence of porcine circovirus DNA: affinities with plant circoviruses. J. Gen. Virol. 1997. **78**. 221-227) a été utilisé comme source d'ADN viral spécifique pour PCV. Un plasmide analogue, pCAA1, contenant la forme réplicative 2,3 kpb du circovirue aviaire CAV a été utilisé comme contrôle négatif. Les stocks de glycérols respectifs de ces deux plasmides ont été utilisés pour la production et la purification des plasmides selon la technique de lyse alcaline (Sambrook J. *et al*. Molecular cloning : A Laboratory Manual. 2^{nd} Edition. Cold Spring Harbor Laboratory. Cold Spring Harbor. New york. 1989) afin qu'ils servent ensuite de matrices pour la préparation des sondes. Les sondes circovirus représentatives des génomes complets du PCV et de CAV ont été produites à partir des plasmides purifiés décrits ci-dessus (1 µg pour chaque sonde) et d'amorces hexanucléotidiques au hasard en utilisant un kit commercial de marquage non radioactif ("DIG DNA labenng kit", Boehringer Mannheim, Lewes, UK) selon les recommandations du fournisseur.

Les sondes marquées à la digoxigénine ont été reprises sous un volume de 50-100 µl d'eau stérile avant leur utilisation pour l'hybridation *in situ*.

Les échantillons de tissus de porcs malades, inclus dans la paraffine et fixés au formaldéhyde, ainsi que les préparations de cultures de cellules infectées, fixées au formaldéhyde, ont été préparées pour la détection des acides nucléiques PCV selon la technique suivante :

Des sections de 5 µm d'épaisseur ont été découpées à partir des blocs de tissus inclus dans la paraffine, déparaffinés, puis réhydratés dans des solutions successives d'alcool à concentration décroissante. Les sections de tissus et les cultures de cellules fixées au formaldéhyde ont été incubées respectivement pendant 16 minutes et 6 minutes à +37°C dans une solution de protéinase K à 0,5% en tampon Tris-HCl 0,05M, EDTA 6 mM (pH 7,6). Les lames ont été alors placées dans une solution de glycine à 1 % en eau distillée autoclavée, pendant 30 secondes, lavées deux fois avec un tampon PBS (phosphate buffer saline) 0,01 M (pH 7,2), et enfin lavées pendant 6 minutes en eau distillée stérile. Elles ont été finalement séchées à l'air libre et mises en contact avec les sondes.

Chaque préparation tissu/sonde a été recouverte avec une lamelle propre et dégraissée, puis placée dans un four à +90°C pendant 10 minutes, mise ensuite en contact avec un bloc de glace pendant 1 minute, et enfin incubée pendant 18 heures à +37°C. Les préparations ont été ensuite immergées brièvement dans un tampon sel de sodium-citrate (SSC) 2X (pH 7,0) pour éliminer les lamelles protectrices, puis lavées 2 fois pendant 5 minutes en tampon SSC 2X et enfin lavées 2 fois pendant 5 minutes en tampon PBS.

Après ces lavages, les préparations ont été immergées dans une solution d'acide maléique 0,1 M, NaCl 0,15 M (pH 7,5) (tampon maléique) pendant 10 minutes, puis incubées dans une solution de 1% de réactif bloquant (Cat # 1096176, Boehringer Mannheim UK, Lewis, East Sussex, UK) en tampon maléique pendant 20 minutes à + 37°C.

Les préparations ont alors été incubées avec une solution au 1/250 d'un anticorps monoclonal anti-digoxigénine (Boehringer Mannheim), dilué en tampon bloquant, pendant 1 heure à +37°C, lavées en PBS et enfin incubées avec un anticorps biotinylé anti-immunoglobuline de souris pendant 30 minutes à + 37°C. Les préparations ont été lavées en PBS et l'activité péroxydase endogène a été bloquée par un traitement avec une solution de péroxyde d'hydrogène à 0,5% en PBS pendant 20 minutes à température ambiante. Les préparations ont été lavées une nouvelle fois en PBS et traitées avec un substrat 3-amino-9-diéthylcarbazole (AEC) (Cambridge Bioscience, Cambridge, UK) préparé extemporanément.

Après un dernier lavage à l'eau de ville, les préparations ont été contre-colorées avec de l'hématoxyline, "bleuies" sous eau de ville, et montées sous lamelles microscopiques avec un liquide de montage (GVA Mount, Cambridge Bioscience, Cambridge, UK). Les contrôles d'expérience ont inclus l'utilisation d'une sonde négative non pertinente (CAV) et d'une sonde positive (PCV) sur des échantillons provenant de porcs malades et de porcs non malades.

### Exemple 4 : Technique de détection du PCV par immunofluorescence

Le criblage initial de toutes les préparations de culture cellulaire fixées à l'acétone a été réalisé par une technique d'immunofluorescence indirecte (IFI) utilisant une dilution au 1/100 d'un pool de sérums de porcs adultes. Ce pool de sérums comprend des sérums de 25 truies adultes d'lrlande du Nord et est connu pour contenir des anticorps contre une grande variété de virus porcins, y compris PCV : parvovirus porcin, adénovirus porcin, et virus PRRS. La technique IFI a été réalisée par un contact du sérum (dilué en PBS) avec les cultures cellulaires pendant une heure à +37°C, suivi de deux lavages en PBS. Les cultures de cellules sont alors colorées avec une dilution au 1/80 en PBS d'un anticorps de lapin anti-immunoglobuline de porc conjugué à de l'isothiocyanate de fluorescéine pendant une heure, puis lavées en PBS et montées en tampon glycérol préalablement à l'observation microscopique sous éclairage ultra-violet.

### Exemple 5 : Résultats de l'hybridation in situ sur les tissus de porcs malades

L'hybridation *in situ*, utilisant une sonde génomique PCV, réalisée sur des tissus prélevés sur des porcelets français, canadiens et californiens présentant des lésions de dépérissement généralisé et fixés au formaldéhyde, a révélé la présence d'acides nucléiques PCV associés aux lésions, dans plusieurs des lésions étudiées. Aucun signal n'a été observé lorsque la sonde génomique PCV a été utilisée sur des tissus prélevés sur des porcs non malades ou lorsque la sonde CAV a été utilisée sur les tissus de porcs malades. La présence d'acide nucléique PCV a été identifiée dans le cytoplasme et le noyau de nombreuses cellules mononucléaires infiltrant les lésions dans les poumons des porcelets californiens. La présence d'acide nucléique PCV a également été mise en évidence dans les pneumocytes, les cellules épithéliales bronchiques et bronchiolaires, et dans les cellules endothéliales des petites artérioles, veinules et vaisseaux lymphatiques.

Chez les porcs malades français, la présence d'acide nucléique PCV a été détectée dans le cytoplasme de nombreux lymphocytes folliculaires et dans les cellules mononucléaires intrasinusoïdales des ganglions lymphatiques. L'acide nucléique PCV a également été détecté dans des syncytia occasionnels. En fonction de ces résultats de détection, des échantillons de poumons de porcs californiens, de ganglions lymphatiques mésentériques de porcs français, et d'organes de porcs canadiens ont été choisis aux fins d'isolement des nouvelles souches de circovirus porcin.

### Exemple 6 : Résultats de la culture cellulaire des nouvelles souches de circovirus porcin et détection par immunofluorescence

Aucun effet cytopathique (ECP) n'a été observé dans les cultures de cellules inoculées avec les échantillons prélevés sur les porcelets francais (souche lmp.1008), californiens (souche Imp.999) et canadiens (souche Imp.1010) montrant des signes cliniques du syndrome du dépérissement généralisé. Cependant, l'immuno-marquage des préparations provenant des cultures de cellules inoculées, après fixation à l'acétone et avec un pool de sérums polyclonaux de porcs, a révélé une fluorescence nucléaire chez de nombreuses cellules dans les cultures inoculées à partir des poumons de porcelets californiens (souche lmp.999), à partir des ganglions lymphatiques médiastinaux des porcelets français (souche lmp.1008), et à partir d'organes des porcelets canadiens (souche Imp.1010).

### Exemple 7 : Extraction de l'ADN génomique des circovirus porcins

Les formes réplicatives des nouvelles souches de circovirus porcin (PCV) ont été préparées à partir de cultures de cellules PK/15 infectées (voir exemple 1) (10 Falcons de 75 cm²) récoltées après 72-76 heures d'incubation et traitées à la glucosamine, comme décrit pour le clonage de la forme réplicative du CAV (Todd. D. *et al*. Dot blot hybridization assay for chicken anemia agent using a cloned DNA probe. J. Clin. Microbiol. 1991. **29**. 933-939). L'ADN double brin de ces formes réplicatives a été extrait selon une modification de la technique de Hirt (Hirt B. Selective extraction of polyoma virus DNA from infected cell cultures. J. Mol. Biol. 1967. **36.** 365-369), comme décrit par Molitor (Molitor T.W. *et al*. Porcine parvovirus DNA: characterization of the genomic and replicative form DNA of two virus isolates. Virology. 1984. **137**. 241-254).

### Exemple 8 : Carte de restriction de la forme réplicative du génome de la souche Imp.999 de circovirus porcin.

L'ADN (1-6 µg) extrait selon la technique de Hirt a été traité par la nucléase S1 (Amersham) selon les recommandations du fournisseur, puis cet ADN a été digéré par différentes enzymes de restriction (Boehringer Mannheim, Lewis, East Sussex, UK) et les produits de la digestion ont été séparés par électrophorèse sur gel d'agarose à 1,5% en présence de bromure d'éthidium comme décrit par Todd *et al*. (Purification and biochemical characterization of chicken anemia agent. J. Gen. Virol. 1990. 71. 819-823).
L'ADN extrait des cultures de la souche lmp.999 possède un site unique EcoRI, 2 sites Sacl et ne possède pas de site Pstl. Ce profil de restriction est donc différent du profil de restriction présenté par la souche PCV PK/15 (Meehan B. *et al.* Sequence of porcine circovirus DNA: affinities with plant circoviruses. 1997. **78.** 221-227) qui possède au contraire un site Pstl et ne possède pas de site EcoRI.

### Exemple 9 : Clonage du génome de la souche lmp.999 de circovirus porcin

Le fragment de restriction d'environ 1,8 kpb généré par digestion de la forme réplicative double brin de la souche PCV lmp.999 avec l'enzyme de restriction EcoRI a été isolé après électrophorèse sur gel d'agarose à 1,5% (voir exemple 3) en utilisant un kit commercial Qiagen (QIAEXII Gel Extraction Kit, Cat # 20021,QIAGEN Ltd., Crawley, West Sussex, UK). Ce fragment de restriction EcoRI-EcoRI a été ensuite ligaturé avec le vecteur pGEM-7 (Promega, Medical Supply Company, Dublin, Ireland), préalablement digéré par les mêmes enzymes de restriction et déphosphoryté, en suivant les techniques standards de clonage (Sambrook J. *et al*. Molecular cloning : A Laboratory Manual. 2^{nd} Ecrdion. Cold Spring Harbor Laboratory. Cold Spring Harbor. New york. 1989). Les plasmides obtenus ont été transformés dans une souche hôte *Escherichia coli* JM109 (Stratagene, La Jolla, USA) selon les techniques standards. Le fragment de restriction EcoRI-EcoRI de la souche PCV Imp.999 a également été cloné dans le site EcoRI du vecteur pBlueScript SK+ (Stratagene Inc. La Jolla, USA). Parmi les clones obtenus pour chaque souche hôte, au moins 2 clones contenant les fragments de la taille attendue ont été sélectionnés. Les clones obtenus ont alors été cultivés et les plasmides contenant le génome complet de la souche lmp.999 ont été purifiés en petit volume (2 ml) ou en grand volume (250 ml) selon les techniques standards de préparation et de purification des plasmides.

### Exemple 10 : Séquençage de l'ADN génomique (forme réplicative double brin) de la souche PCV Imp.999.

La séquence nucléotidique de 2 clones EcoRI lmp.999 (clones pGEM-7/2 et pGEM-7/8) a été déterminée selon la technique des didéoxynucléotides de Sanger en utilisant le kit de séquençage "AmpliTaq DNA polymerase FS" (Cat # 402079 PE Applied Biosystems, Warrington, UK) et un appareil de séquençage automatique Applied BioSystems AB1373A selon les recommandations du fournisseur. Les réactions de séquençage initiales ont été faites avec les primers universels M13 "forward" et "reverse". Les réactions de séquençage suivantes ont été générées selon la technique de "marche sur l'ADN". Les oligonucléotides nécessaires à ces séquençages ultérieurs ont été synthétisés par Life Technologies (Inchinnan Business Park, Paisley, UK).

Les séquences générées ont été assemblées et analysées au moyen du logiciel MacDNASIS version 3.2. (Cat # 22020101, Appligene, Durham, UK). Les différents cadres ouverts de lecture ont été analysés au moyen de l'algorithme BLAST disponible sur le serveur du "National Center for Biotechnology Information (NCBI, Bethesda, MD, USA).

La séquence complète (fragment EcoRI-EcoRI) obtenue initialement à partir du clone pGEM-7/8 (SEQ ID NO : 6) est présentée sur la figure N°6. Elle débute arbitrairement après le G du site EcoRl et présente quelques incertitudes sur le plan des nucléotidiques.

Le séquençage a ensuite été optimisé et la SEQ ID NO : 3 (Figure 3) donne la séquence totale de cette souche, que l'on a fait débuté arbitrairement au début du site EcoRI, soit le G comme premier nucléotide.

On a procédé d'une manière similaire pour l'obtention de la séquence des trois autres isolats selon l'invention (voir SEQ ID NO : 1, 2 et 4 et figures 1, 2 et 4).

La taille du génome de ces quatre souches est :

| | |
|---|---|
| Imp 1011-48121 | 1767 nucléotides |
| Imp 1011-48285 | 1787 nucléotides |
| Imp 999 | 1768 nucléotides |
| Imp 1010 | 1788 nucléotides |

### Exemple 11 : Analyse de la séquence de la souche PCV Imp.999.

Lorsque la séquence générée à partir de la souche lmp.999 a été utilisée pour une recherche d'homologie vis-à-vis des séquences contenues dans la banque de données GenBank, la seule homologie significative qui ait été détectée est une homologie d'environ 76 % (au niveau acide nucléique) avec la séquence de la souche PK/15 (Numéros d'accès Y09921 et U49186) (voir figure N°5).

Au niveau acides aminés, la recherche d'homologie de la traduction des séquences dans les 6 phases avec les banques de données (algorithme BLAST X sur le serveur NCBI) a permis de mettre en évidence une homologie de 94 % avec le cadre ouvert de lecture correspondant à la réplicase théorique du virus BBTV similaire aux circovirus de plantes (numéro d'identification GenBank 1841515) codée par la séquence GenBank U49186.

Aucune autre séquence contenue dans les banques de données ne montre d'homologie significative avec la séquence générée à partir de la souche PCV Imp.999.

L'analyse des séquences obtenues à partir de la souche lmp.999 cultivée à partir de lésions prélevées sur des porcelets californiens présentant des signes cliniques du syndrome de dépérissement généralisé montre clairement que cet isolat viral est une nouvelle souche de circovirus porcin.

### Exemple 12 : Analyse comparative des séquences

L'alignement des séquences nucléotidiques des 4 nouvelles souches PCV a été fait avec la séquence de la souche PCV PK/15 (figure 5). Une matrice d'homologie prenant en compte les quatre nouvelles souches et la souche antérieure PK/15 a été réalisée. Les résultats sont les suivants :
1 : lmp 1011-48121
2 : Imp 1011-48285
3 : lmp 999
4 : Imp 1010
6 : PK/15

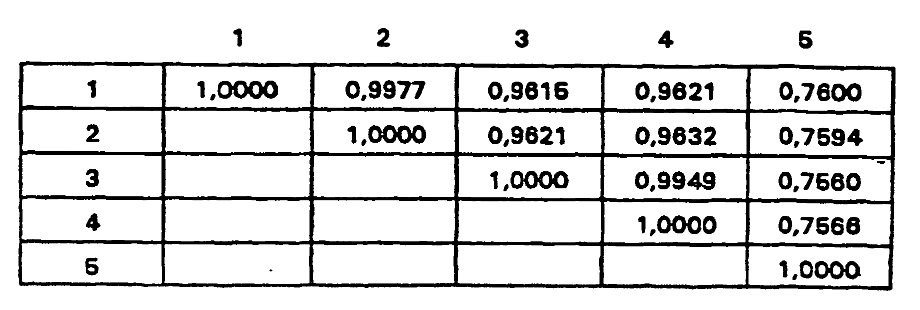

L'homologie entre les deux souches françaises lmp 1011-48121 et Imp 1011-48285 est supérieure à 99 % (0,9977).

L'homologie entre les deux souches nord-américaines lmp 999 et lmp 1010 est aussi supérieure à 99 % (0,9949). L'homologie entre souches françaises et souches nord-américaines est un peu supérieure à 96 %.

L'homologie de toutes ces souches avec PK/15 tombe à une valeur comprise entre 76 et 76 %.

On en déduit que les souches selon l'invention sont représentatives d'un nouveau type de circovirus porcin, distinct du type représenté par la souche PK/15. Ce nouveau type, isolé de porcs présentant le syndrome PMWS, est dénommé circovirus porcin de type II, la PK/16 représentant le type I. Les souches appartenant à ce type II présentent une remarquable homogénéité de séquence nucléotidique, alors même qu'elles ont été isolées dans des régions géographiques très éloignées.

### Exemple 13 : Analyse des protéines codées par le génome des nouvelles souches PCV.

La séquence nucléotidique de l'isolat Imp. 1010 a été considérée comme représentative des autres souches de circovirus associées au syndrome de dépérissement généralisé. Cette séquence a été analysée plus en détail à l'aide de l'algorithme BLASTX (Altschul *et al.* J. Mol. Biol. 1990. **215.** 403-410) et d'une combinaison de programmes de l'ensemble de logiciels MacVector 6.0 (Oxford Molecular Group, Oxford OX4 4GA, UK). Il a été possible de détecter 13 cadres ouverts de lecture (ou COLs) d'une taille supérieure à 20 acides aminés sur cette séquence (génome circulaire). Ces 13 COLs sont les suivants :

| Nom | Début | Fin | Brin | Taille du COL (nucléotides (nt)) | Taille protéine (acides aminés (aa)) |
|---|---|---|---|---|---|
| COL1 | 103 | 210 | sens | 108 nt | 35 aa |
| COL2 | 1180 | 1317 | sens | 138 nt | 45 aa |
| COL3 | 1363 | 1524 | sens | 162 nt | 53 aa |
| COL4 | 398 | 1342 | sens | 945 nt | 314 aa |
| COL5 | 900 | 1079 | sens | 180 nt | 59 aa |
| COL6 | 1254 | 1334 | sens | 81 nt | 26 aa |
| COL7 | 1018 | 704 | antisens | 315 nt | 104 aa |
| COL8 | 439 | 311 | antisens | 129 nt | 42 aa |
| COL9 | 190 | 101 | antisens | 90 nt | 29 aa |
| COL10 | 912 | 733 | antisens | 180 nt | 59 aa |
| COL11 | 645 | 565 | antisens | 81 nt | 26 aa |
| COL12 | 1100 | 1035 | antisens | 66 nt | 21 aa |
| COL13 | 314 | 1381 | antisens | 702 nt | 213 aa |

Les positions de début et de fin de chaque COL se réfèrent à la séquence présentée sur la figure N° 4 (SEQ ID N° 4), du génome de la souche 1010. Les limites des COLs 1 à 13 sont identiques pour la souche 999. Elles le sont aussi pour les souches 1011-48121 et 1011-48285, sauf pour les COLs 3 et 13 :
COL3 1432-1539, sens, 108 nt, 35aa
COL13 314-1377, antisens, 705 nt, 234 aa.

Parmi ces 13 COLs, 4 présentent une homologie significative avec des COLs analogues situés sur le génome du virus cloné PCV PK-15. Chacun des cadres ouverts de lecture présents sur le génome de tous les isolats de circovirus associés au syndrome de dépérissement généralisé a été analysé. Ces 4 COLs sont les suivants :

| Nom | Début | Fin | Brin | Taille du COL (nt) | Taille protéine (acides aminés) | Masse moléculaire |
|---|---|---|---|---|---|---|
| COL4 | 398 | 1342 | sens | 945 nt | 314 aa | 37,7 kDa |
| COL7 | 1018 | 704 | antisens | 315 nt | 104 aa | 11,8 kDa |
| COL10 | 912 | 733 | antisens | 180 nt | 59 aa | 6,5 kDa |
| COL13 | 314 | 1381 | antisens | 702 nt | 233 aa | 27,8 kDa |

Les positions de début et de fin de chaque COL se référent à la séquence présentée sur la figure N° 4 (SEQ ID N° 4). La taille du COL (en nucléotides = nt) inclut le codon stop.

La comparaison entre l'organisation génomique des isolats PCV lmp. 1010 et PCV PK-15 a permis l'identification de 4 COLs conservés dans le génome des deux virus. Le tableau ci-dessous présente les degrés d'homologie observés:

| **COL Imp. 1010/COL PCV PK-15** | **Pourcentage d'homologie** |
|---|---|
| COL4/COL1 | 86 % |
| COL13/COL2 | 66,4 % |
| COL7/COL3 | 61,5 % (au niveau du recouvrement (104 aa) |
| COL10/COL4 | 83 % (au niveau du recouvrement (59 aa) |

La plus grande identité de séquence a été observée entre le COL4 lmp. 1010 et le COL1 PK-15 (86% d'homologie). Ceci était attendu dans la mesure où cette protéine est probablement impliquée dans la réplication de l'ADN viral et est essentielle pour la réplication virale (Meehan *et al.* J. Gen. Virol.1997. **78**. 221-227; Mankertz *et al*. J. Gen. Virol. 1998. **79**. 381-384).

L'identité de séquence entre le COL13 lmp. 1010 et le COL2 PK-15 est moins forte (66,4% d'homologie), mais chacun de ces deux COLs présente bien une région basique N-terminale très conservée, qui est identique à la région N-terminale de la protéine structurale majeure du circovirus aviaire CAV (Meehan *et al.* Arch. Virol. 1992. **124.** 301-319). De plus grandes différences sont observées entre COL7 lmp. 1010 et COL3 PK-15 et entre COL10 Imp. 1010 et COL4 PK-15. Dans chaque cas, il existe une délétion de la région C-terminale des COL7 et COL10 de l'isolat lmp. 1010 lorsqu'on les compare aux COL3 et COL4 de PCV PK-15. La plus haute homologie de séquence est observée au niveau des régions N-terminales de COL7/COL3 (61,5% d'homologie au niveau du recouvrement) et de COL10/COL4 (83% d'homologie au niveau du recouvrement).

Il apparaît que l'organisation génomique du circovirus porcin est assez complexe suite à l'extrême compacité de son génome. La protéine structurale majeure est probablement issue d'un épissage entre plusieurs cadres de lecture situés sur le même brin du génome du circovirus porcin. On peut donc considérer que tout cadre ouvert de lecture (COL1 à COL13) tel que décrit dans le tableau ci-dessus, peut représenter tout ou partie d'une protéine antigénique codée par le circovirus porcin de type II et est donc potentiellement un antigène utilisable pour le diagnostic spécifique et/ou pour la vaccination. L'invention concerne donc toute protéine comprenant au moins un de ces COLs. De préférence, l'invention concerne une protéine formée essentiellement par les COL4, COL7, COL10 ou COL13.

### Exemple 14 : Caractère infectieux du génome PCV cloné à partir des nouvelles souches.

Le plasmide pGEM-7/8 contenant le génome complet (forme réplicative) de l'isolat Imp.999 a été transfecté dans des cellules PK/15 selon la technique décrite par Meehan B. *et al*. (Characterization of viral DNAs from cells infected with chicken anemia agent : sequence analysis of the cloned replicative form and transfection capabilities of cloned genome fragments. Arch. Virol. 1992. **124**. 301-319). L'analyse par immunofluorescence (voir exemple 4) réalisée sur le premier passage après bansfection sur cellules PK/15 non contaminées a montré que le plasmide du clone pGEM7/8 était capable d'induire la production de virus PCV infectieux. La disponibilité d'un clone contenant un matériel génétique PCV infectieux permet toute manipulation utile sur le génome viral afin de produire des virus PCV modifiés (soit atténués chez le porc, soit défectifs) utilisables pour la production de vaccins atténués ou recombinés, ou pour la production d'antigènes pour des trousses de diagnostic.

### Exemple 15 : Production des antigènes PCV par culture in vitro

La culture des cellules PK/15 non contaminées et la multiplication virale sont réalisées selon les mêmes modalités qu'à l'exemple 1. Les cellules infectées sont récoltées après trypsination après 4 jours d'incubation à 37 °C et numérées. Le passage suivant est inoculé avec 400 000 cellules infectées par ml.

### Exemple 16 : Inactivation des antigènes viraux

En fin de culture virale, les cellules infectées sont récoltées et lysées par ultrasons (Branson Sonifier) ou à l'aide d'un broyeur colloïdal de type rotor-stator. (UltraTurrax, IKA). La suspension est ensuite centrifugée à 3700 g pendant 30 minutes. La suspension virale est inactivée par 0,1 % d'éthylène imine pendant 18 heures à +37°C ou par 0,5 % de bêta-propiolactone pendant 24 heures à +28°C. Si le titre du virus avant inactivation est insuffisant, la suspension virale est concentrée par ultrafiltration en utilisant une membrane avec un seuil de coupure de 300 kDa (Millipore PTMK300). La suspension virale inactivée est conservée à +5°C.

### Exemple 17 : Préparation du vaccin sous forme d'émulsion à base d'huile minérale.

Le vaccin est préparé selon la formule suivante :
- suspension de circovirus porcin inactivé : 250 ml
- Montanide® ISA 70 (SEPPIC): 750 ml
La phase aqueuse et la phase huileuse sont stérilisées séparément par filtration. L'émulsion est préparée par mélange et homogénéisation des ingrédients à l'aide d'un émulseur à turbine Silverson.

Une dose de vaccin contient environ 10^{7,8} DICT50. Le volume d'une dose de vaccin est de 0,5 ml pour administration par voie intradermique, et de 2 ml pour administration par voie intramusculaire.

### Exemple 18 : Préparation du vaccin sous forme d'émulsion à base d'huile métabolisable.

Le vaccin est préparé selon la formule suivante :
- suspension de circovirus porcin inactivé : 200 ml
- Dehymuls HRE 7 (Henkel) : 80 ml
- Radia 7204 (Oleofina) : 740 ml

La phase aqueuse et la phase huileuse sont stérilisées séparément par filtration. L'émulsion est préparée par mélange et homogénéisation des ingrédients à l'aide d'un émulseur à turbine Silverson.

Une dose de vaccin contient environ 10^{7,6} DICT50. Le volume d'une dose de vaccin est de 2 ml pour administration par voie intramusculaire.

### Exemple 19 : Résultats d'immunofluorescence indirecte vis-à-vis des souches de virus PCV US et française et du contaminant PK/15 avec un sérum hyperimmun (PCV-T), un panel d'anticorps monocionaux F99, préparés à partir de PK/15 et un sérum hyperimmun préparé à partir de la souche canadienne (PCV-C)

| VIRUS | | | |
|---|---|---|---|
| | PK/15 | USA | France |
| PCV-T antiserum | ≥ 6 400 | 200 | 800 |
| PCV-C antiserum | 200 | ≥ 6,400 | ≥ 6,400 |
| F99 1H4 | ≥ 10 000 | <100 | 100 |
| F99 4B10 | ≥ 10 000 | <100 | < 100 |
| F99 287 | ≥ 10 000 | 100 | < 100 |
| F99 2E12 | ≥ 10 000 | <100 | <100 |
| F99 1C9 | ≥ 10 000 | <100 | 100 |
| F99 2E1 | ≥ 10 000 | <100 | <100 |
| F99 1H4 | ≥ 10 000 | 100 | <100 |
| * inverse de la dernière dilution du sérum ou de l'anticorps monoclonal qui donne une réaction positive en immunofluorescence indirecte. | | | |

## Revendications

1. Circovirus porcin de type II isolé responsable chez le porc du syndrome de dépérissement généralisé de post-sevrage (PMWS).

2. Circovirus selon la revendication 1, susceptible d'être isolé d'un porc malade présentant le syndrome PMWS.

3. Circovirus selon la revendication 1 ou 2, tel que déposé auprès de l'ECACC sous l'une des références suivantes :
- V97100219
- V97100218
- V97100217
- V98011608
- V98011609.

4. Préparation de circovirus porcin de type II isolé responsable chez le porc du syndrome de dépérissement généralisé de post-sevrage (PMWS), issue de culture cellulaire.

5. Préparation selon la revendication 4, dans laquelle le circovirus porcin de type II est susceptible d'être isolé d'un porc malade présentant le syndrome PMWS.

6. Préparation selon la revendication 4 ou 5, choisie dans le groupe consistant dans les préparations déposées auprès de l'ECACC sous l'une des références suivantes :
- V97100219
- V97100218
- V97100217
- V98011608
- V98011609.

7. Préparation selon l'une quelconque des revendications 4 à 6, purifiée.

8. Préparation selon l'une quelconque des revendications 4 à 7, concentrée.

9. Préparation selon l'une quelconque des revendications 4 à 8, inactivée.

10. Procédé de production de circovirus porcin de type II responsable chez le porc du syndrome de dépérissement généralisé de post-sevrage (PMWS), dans lequel des cellules en culture cellulaire *in vitro* sont infectées avec un circovirus selon l'une quelconque des revendications 1 à 3, et en ce que ledit circovirus se multiplie sur ces cellules.

11. Procédé selon la revendication 10, dans lequel, comme cellules, on utilise des cellules d'une lignée cellulaire de rein de porc.

12. Procédé selon la revendication 11, dans lequel l'on utilise des cellules PK/15.

13. Procédé selon l'une quelconque des revendications 10 à 12, comprenant la récolte et la purification du circovirus porcin de type II.

14. Procédé selon l'une quelconque des revendications 10 à 13, comprenant la concentration du circovirus porcin de type II.

15. Procédé selon l'une quelconque des revendications 10 à 14, comprenant l'inactivation du circovirus porcin de type II.

16. Extrait ou surnageant de culture contenant un circovirus porcin de type II et susceptible d'être obtenu par mise en oeuvre du procédé selon l'une des revendications 10 à 12.

17. Préparation antigénique contenant un circovirus porcin de type II et susceptible d'être obtenue par mise en oeuvre du procédé selon l'une des revendications 10 à 15.

## Patentansprüche

1. Isoliertes porcines Circovirus Typ II, das beim Schwein für das PMWS-Syndrom ("Post Weaning Multisystemic Wasting Syndrome") verantwortlich ist.

2. Circovirus nach Anspruch 1, das von einem kranken Schwein, das das PMWS-Syndrom aufweist, isoliert werden kann

3. Circovirus nach Anspruch 1 oder 2, das beim ECACC unter den folgenden Nummern hinterlegt ist
- V97100219
- V97100218
- V97100217
- V98011608
- V98011609

4. Isoliertes porcines Circovirus-Typ-II-Praparat, das beim Schwein fur das PMWS-Syndrom ("Post Weaning Multisystemic Wasting Syndrome") verantwortlich ist und aus einer Zellkultur stammt.

5. Präparat nach Anspruch 4, wobei das porcine Circovirus Typ II von einem kranken Schwein, das das PMWS-Syndrom aufweist, isoliert werden kann

6. Präparat nach Anspruch 4 oder 5, ausgewahlt aus der Gruppe bestehend aus den bei der ECACC unter folgenden Nummern hinterlegten Präparaten:
- V97100219
- V97100218
- V97100217
- V98011608
- V98011609.

7. Gereinigtes Präparat nach einem der Ansprüche 4 bis 6.

8. Konzentriertes Präparat nach einem der Ansprüche 4 bis 7.

9. Inaktiviertes Präparat nach einem der Ansprüche 4 bis 8.

10. Herstellungsverfahren fur porcines Circovirus Typ II, das beim Schwein für das PMWS-Syndrom verantwortlich ist, wobei die Zellen in Zellkultur *in vitro* mit einem Circovirus nach einem der Anspruche 1 bis 3 infiziert sind und wobei sich der Circovirus auf den Zellen vervielfältigt.

11. Verfahren nach Anspruch 10, bei dem als Zellen Zellen aus einer Schweinenierenzelllinie verwendet werden

12. Verfahren nach Anspruch 11, in dem PK/15-Zellen verwendet werden.

13. Verfahren nach einem der Anspruche 10 bis 12, umfassend die Gewinnung und die Reinigung des porcinen Circovirus Typ II

14. Verfahren nach einem der Anspruche 10 bis 13, umfassend das Konzentrieren des porcinen Circovirus Typ II

15. Verfahren nach einem der Anspruche 10 bis 14, umfassend die Inaktivierung des porcinen Circovirus Typ II

16. Kulturextrakt oder -überstand, der ein porcines Circovirus Typ II enthalt, das durch die Durchfuhrung des Verfahrens nach einem der Anspruche 10 bis 12 erhalten werden kann

17. Antigenes Präparat, das ein porcine Circovirus Typ II enthalt, das durch die Durchfuhrung des Verfahrens nach einem der Anspruche 10 bis 15 erhalten werden kann.

## Claims

1. Isolated type II porcine circovirus responsible for Post-Weaning Multisystemic Wasting Syndrome (PMWS) in pigs

2. Circovirus according to claim 1, isolatable from a diseased pig suffenng from PMWS

3. Circovirus according to claim 1 or 2, as deposited with the ECACC under one of the following references:
- V97100219
- V97100218
- V97100217
- V98011608
- V98011609

4. Preparation of isolated type II porcine circovirus responsible for Post-Weaning Multisystemic Wasting Syndrome (PMWS) in pigs, obtained from cultured cells

5. Preparation according to claim 4, wherein said type II porcine circovirus is isolatable from a diseased pig suffering from PMWS.

6. Preparation according to claim 4 or 5, selected from the group consisting of the preparations deposited with the ECACC under one of the following references:
- V97100219
- V97100218
- V97100217
- V98011608
- V98011609.

7. Preparation according to any one of claims 4-6, which is punfied.

8. Preparation according to any one of claims 4-7, which is concentrated.

9. Preparation according to any one of claims 4-8, which is inactivated.

10. Process for preparing a type II porcine circovirus responsible for Post-Weaning Multisystemic Wasting Syndrome (PMWS) in pigs, wherein cells cultured in vitro are infected with a circovirus according to any one of claims 1-3, and wherein said circovirus multiplies on said cells.

11. Process according to claim 10, wherein the cells used are cells from a pig kidney cell line.

12. Process according to claim 11, wherem the cells used are PKL/15 cells.

13. Process according to any one of claims 10-12, comprising harvesting and purifying said type II porcine circovirus.

14. Process according to any one of claims 10-13, comprising concentrating said type II porcine circovirus.

15. Process according to any one of claims 10-14, comprising inactivating said type II porcine circovirus.

16. Culture supernatant or extract containing a type II porcine circovirus, obtainable via the process according to one of claims 10-12.

17. Antigemc preparation containing a type II porcine circovirus obtainable via the process according to one of claims 10-15.
